# EUROPEAN PATENT APPLICATION

(11) **EP 4 389 174 A1**
(43) Date of publication of application: **26.06.2024**
(21) Application number: 22306961.8
(22) Date of filing: 20.12.2022
(51) Int. Cl.: A61M 5/28, A61M 5/31, A61M 5/32

(54) **SAFETY DEVICE FOR MOUNTING ONTO A DRUG DELIVERY DEVICE**

(71) Applicant: Becton Dickinson France, 38800 Le Pont-de-Claix (FR)
(72) Inventor: ALBENGE, Olivier, 38130 Echirolles (FR); CHANSAVANG, Albert, 38000 Grenoble (FR); MILLS, Freddy, 38000 Grenoble (FR)
(74) Representative: Germain Maureau

(57) **Abstract**

The safety device (10,210) for mounting onto a drug delivery device (100) having a barrel (102) with a flange (107), an injection needle (105), and a piston rod (108) with a flange (109), includes a tubular body (12) configured to connect to the barrel (102), a safety member (20, 220) disposed within the tubular body (12) configured to occupy a resting state where the safety member (20,220) does not extend distally from the tubular body (12) and a safety state where the safety member (20,220) shields the needle (105), said safety member (20,220) having an actuation member (22, 222) configured to be pushed by the piston flange (109) and a compressive member (21,221) connected to the actuation member (22,222), a locking member configured to engage with the safety member (20,220) to maintain the compressive member (20, 220) within the tubular body (12) and to axially release the compressive member (21,221) so that the safety member (20,220) can reach its safety state.

## Description

The present invention relates to a safety device for mounting onto a drug delivery device such as a prefilled or pre-fillable syringe in order to protect a user from needle stick injuries after injection of a medical product. The invention also relates to a drug delivery device including this safety device.

### Background

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or drug delivery device of the invention and the "proximal direction" is to be understood as meaning the opposite direction to said direction of injection, that is to say the direction towards the user's hand.

Drug delivery devices, such as pre-fillable or prefilled syringes, usually comprise a hollow body or barrel forming a container for a medical product. This body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

In order to minimize the risks of needle stick injuries, drug delivery devices may be equipped with a safety device that protects the needle after injection.

Although known safety devices are generally satisfactory, they do not always meet all of the user's expectations and they can include a number of components. Therefore, they can be expensive to manufacture and thus this can limit the widespread use of safety devises at the expense of the security of medical users and patients.

They also tend to include components made of various materials, which can be complex and expensive to recycle.

### Summary

A need exists for an improved safety that provides a user with more convenient safety device that is also cost effective and easy to manufacture.

In one aspect, the invention concerns a safety device for mounting onto a drug delivery device having a barrel including with a flange at its proximal end and an injection needle at its distal end, and a piston rod having a piston flange, the safety device including:
a tubular body configured to connect to the barrel,
a safety member at least partially disposed within the tubular body configured to occupy a resting state where the safety member does not extend distally from the tubular body and a safety state where the safety member extend distally and shields the needle, said safety member having an actuation member configured to be pushed by the piston flange and a compressive member connected to the actuation member, said compressive member configured to have a spring action,
a locking arrangement configured to maintain the compressive member within the tubular body when the piston flange exerts a force on the actuation member and configured to axially release the compressive member so that the safety member can reach its safety state.

In some embodiments, the actuation member includes at least one blade rigidly connected to the compressive member.

In some embodiments, the safety device includes at least one hook member axially and proximally projecting from the tubular body.

In some embodiments, the safety device includes an internal rib, which radially projects from the tubular body.

In some embodiments, the at least one hook member being comprised of a resilient tab and a sloped ramp surface.

In some embodiments, the tubular body includes at least one axial groove configured to guide the at least one blade.

In some embodiments, the compressive member includes a series of compressible tubular bellows.

In some embodiments, the compressive member includes at least one strip of compressive bellows.

In some embodiments, the safety member includes a tubular shield connected to the distal end of the compressive member.

In some embodiments, the safety members includes a passive locking arrangement which releases the compressive member when the piston rod has reached its final distal position.

In some embodiments, the safety device includes at least one locking tab connected to the tubular body by a frangible link configured and sized to break when the piston rod reaches its final distal position and compresses the compressive member so that the energy accumulated in the compressive member breaks the frangible link.

In some embodiments, the compressive member includes a least one radially outwardly oriented teeth and the tubular body includes a complementary inwardly oriented teeth configured and sized to abut against the compressive member teeth and retain the compressive member until the energy accumulated in the compressive member makes the compressive member teeth overcome the tubular body teeth.

In some embodiments, the tubular body includes at least one inwardly oriented projection, which configured and sized to retain the compressive member until the energy accumulated in the compressive member makes the compressive member overcome at least one projection.

In some embodiments, the safety member includes a user actionable locking arrangement, which releases the compressive member when the piston rod has reached its final distal position.

In some embodiments, the safety member includes a tilting tab having an inwardly oriented locking lug configured to pivot between a locking position where the locking lug axially locks the compressive member and a release position where the locking lug opens the way for the compressive member.

In another aspect, the invention concerns a drug delivery device having a barrel including with a flange at its proximal end and an injection needle at its distal end, and a piston rod having a piston flange fitted with a safety device as above described.

### Brief description of the drawings

With reference to the appended drawings, below follows a more detailed description of aspects and embodiments of the invention cited as examples.
Fig.1 is exploded view of a safety device according to one embodiment of the invention mounted on a drug delivery device;
Fig.2 is a perspective view of the safety device of Fig.1;
Fig.3 is a perspective view of the safety device of Fig.1;
Fig.4 is a perspective view in cross section of the safety device of Fig.1 in its resting state;
Fig.5 is a side view in cross section view of the safety device of Fig.1 in its resting state;
Fig.6 is a side view in cross section view of the safety device of Fig.1 in during injection;
Fig.7 is a side view in cross section view of the safety device of Fig.1 in its safety state;
Fig.8 is a side view in cross section view of the distal end of the safety device showing an embodiment of a locking arrangement;
Fig.9 is a cross section view of the distal end of the safety device showing an embodiment of a locking arrangement;
Fig.10 is a cross section view of the distal end to the safety device showing an embodiment of a locking arrangement;
Fig.11 is an exploded view of a safety device according to another embodiment of the invention mounted on a drug delivery device;
Fig.12 is a perspective view of the safety device of Fig.11;
Fig.13 is a perspective view in cross section of the safety device of Fig.11;
Fig.14 is a side view in cross section of the safety device of Fig.11 in its resting state;
Fig.15 is a side view in cross section of the safety device of Fig.11 during injection;
Fig.16 is a side view in cross section of the safety device of Fig.11 in its safety state;
Fig.17 is side view in cross section of the distal end of the safety showing an embodiment of a locking arrangement.

### Description of the invention

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

The drug delivery device 100 may be a prefilled or prefillable syringe. The drug delivery device 100 includes a tubular barrel 102 defining a reservoir for containing a medical product such as a drug. The tubular barrel 102 may be made of a plastic or of a glass material. This barrel 102 has a distal shoulder 103 provided with a distal tip 104 longitudinally protruding from said shoulder 103 along a longitudinal axis A. The distal tip 104 defines an axial passageway in fluid communication with the reservoir and is equipped with the injection needle 105 for injecting the medical product in an injection site. The barrel 102 further includes an opposite opened proximal end provided with a flange 107.

The opened proximal end receives a plunger rod 108 for pushing a stopper 112 located inside the barrel 102 to expel the medical product from the reservoir to the injection site via the distal tip 104 and the injection needle 105. At its proximal end, the plunger rod 108 is provided with a plunger flange 109 whereon a user can apply a thumb.

The drug delivery device 100 may suitably be fitted with a tip cap (not shown) for protecting and sealing the injection needle 105 before injection.

The safety device 10 includes a tubular body 12 and a safety member 20.

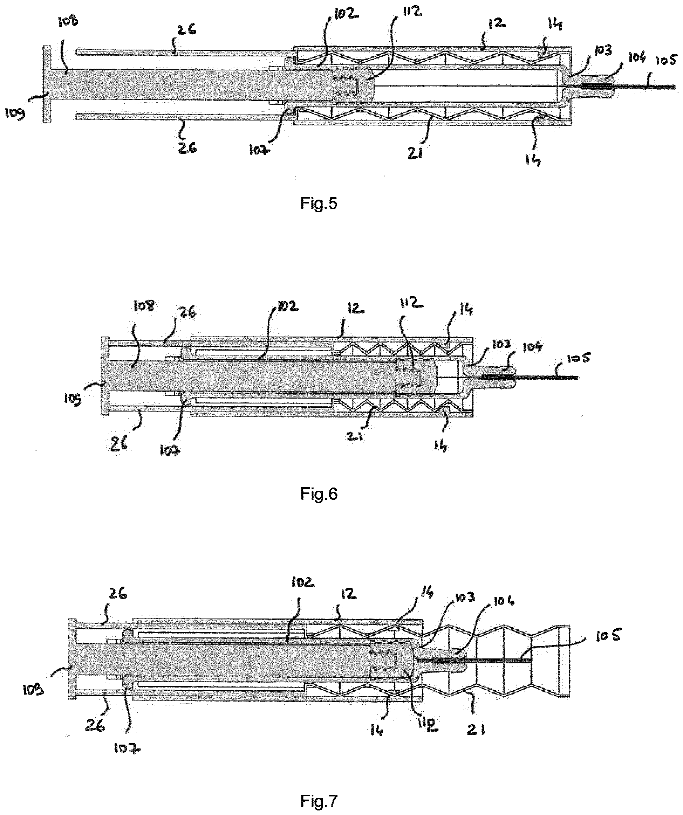

Said tubular body 12 can be axially locked onto the drug delivery device flange 107. Thus, in some embodiments, the safety device 10 can include two hook members 13 axially and proximally projecting from the tubular body 12; each hook member 13 being comprised of a resilient tab 15 and a sloped ramp surface 16. The safety device 10 further includes an internal rib 18, which radially projects from the tubular body 12. The rib 18 is spaced from the sloped ramp surface 16 so that the drug device flange 107 can be locked between the rib 18 and the sloped ramp surface 16. The tubular body 12 may include a finger flange 19 extending radially outwardly. In some embodiments, the tubular body 12 may include two longitudinal grooves 17. As it can be seen on the cross section views of Figs.5 to 7, the tubular body 12 can include one or more projections 14 which extend radially inwardly. In the embodiment illustrated on Figs. 5 to 7, the tubular body 12 includes two projections 14, which radially inwardly extend from the grooves 17. The projections 14 are configured to act as locking arrangement of the safety member 20 within the tubular body 12.

The tubular body 12 may be manufactured by a molding process such as injection molding process using a thermoplastic and/or thermoplastic (TPE) or other disposable and/or recyclable material or combination thereof. Other suitable manufacturing processes may be utilized such as 3D printing.

The safety device 10 further includes the safety member 20. The safety member 20 includes a compressive member 21 and an actuation member 22. In some embodiments, the compressive member 21 includes a series of compressible tubular bellows 24. The term "bellows" as used herein is used to describe a structure possessed by what are traditionally referred to as bellows or accordion or concertina style walls. The term "bellows" also includes a compressible structure achieved by repeated corrugations or bends extending around the circumference or perimeter of a body and has a spring constant that permits expansion and compression of the walls. The compressive member 21 can have an extended position when it is under no axial force and a collapsed position when it is under an axial force. The compressive member 21 can store mechanical energy when it is compressed under an axial force and can resiliently give back the mechanical energy when the force ceases. In some embodiments, the compressive member 21 has a tubular structure and the bellows 24 extend around the circumference of the compressive member 21.

In some embodiments, the actuation member 22 includes two rigid blades 26, which are linked to the compressive member proximal end. The term "rigid" as used herein is used to described a structure that does not bend or flex under a force applied axially. The two blades 26 are spaced apart such that the piston flange 109 can abut against the blades proximal end. The two blades 26 are configured and sized to engage and slide in the grooves 17. The engagement of the blades 26 in the grooves 17 ensures that the safety device does not rotate relative to the drug delivery device 10.

The safety member 22 may be manufactured by a molding process such as injection molding process using a thermoplastic and/or thermoplastic (TPE) or other disposable and/or recyclable material or combination thereof. Other suitable manufacturing processes may be utilized such as 3D printing.

The safety member 22 is engaged in the tubular body 12 to form the safety device 10. The safety member 22 is sized so that the compressive member 24 can be enclosed within the tubular body 12. In some embodiments, the compressive member 24 distal end is flush with the tubular body 12 distal end.

The safety device 10 further includes a locking arrangement configured to maintain the compressive member 21 within the tubular body 12 whether it is in its extended position or in its collapsed position.

The safety device 10 can be fitted on the drug delivery device 100. This can be done by inserting the drug delivery device 100 in the safety device 10. As the engagement progresses, the barrel flange 107 comes in abutment with the hook members 13. As the barrel flange 107 glides on the sloped ramp surfaces 16 making the tabs 15 deflect radially; this allows the barrel flange 107 to snap fit between the annular rib 18 and the hook members 13.

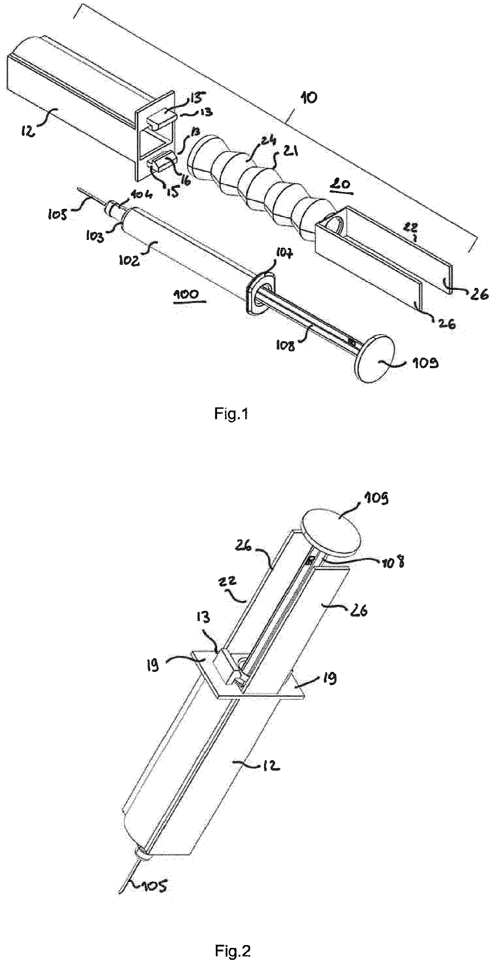

Fig.1 shows the safety device 10 mounted on a drug delivery device 100 in a resting state, which can be, for example, a prefilled syringe.

In Fig.2, the plunger rod 108 is shown moving against the fluid (not shown). As this movement occurs, the piston flange 109 abuts against the blades 26. The blades 26 thus moves axially in the distal direction and bias the compressive member 21.

The compressive member 21 is retained by a locking arrangement.

In some embodiments, the locking arrangement, which retains the compressive member 21 within the tubular body 12, can be passive and requires no action from a user

In the embodiment of Figs. 5 to 7, the projections 14 form a stop, which radially projects between two bellows 24 of the compressive member 21.

Fig.5 shows the safety device 10 in a resting state where the compressive member 21 in its extended position.

Fig.6 shows the safety device 10 in a loaded state during injection as the compressive member 21 is its collapsed position. As the plunger rod 108 moves in the distal direction, the compressive member 21 is compressed against the projection 14. The compressive member 21 is configured and sized so that it acquires an amount of mechanical energy when the plunger rod 108 reaches its final position, said amount of mechanical energy making it possible to overcome the projections 14 thereby releasing the compressive member 21 outside of the tubular body 12 towards its safety position where the compressive member 21 covers the needle 105.

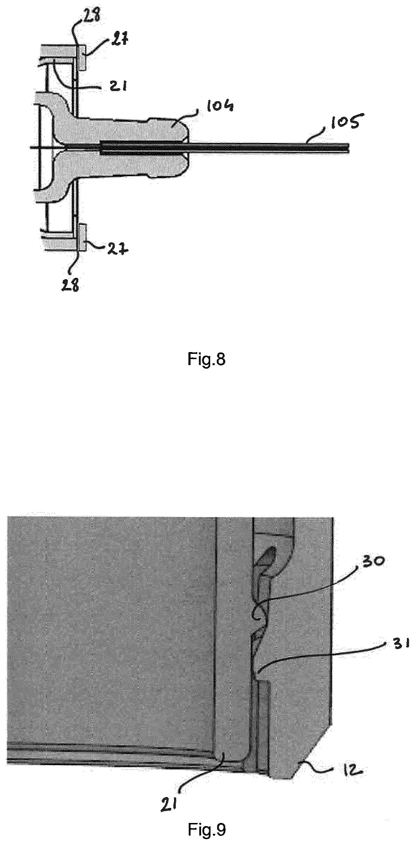

In some embodiments depicted on Fig. 8, the tubular body 12 includes one or more locking tabs 27. The locking tabs 27 are integral with the tubular body distal end and are inwardly radially oriented. The locking tabs 27 can be connected to the tubular body by a frangible link 28, which breaks when a predetermined force is applied. In the illustrated embodiment, the tubular body 12 is provided with two locking tabs 27.

As the plunger rod 108 moves in the distal direction, the compressive member 21 is compressed against locking tabs 27. The frangible links 28 are configured and sized to break when the plunger rod 108 reaches its final position. The amount of mechanical energy accumulated in the compressive member 21 causes a force, which breaks the frangible link 28. Thus, the compressive member 21 is released outside of the tubular body 12 towards its safety position where the compressive member 21 covers the needle 105.

In another embodiment (not shown), the locking tabs 28 flex or elastically deflect to disengage the compressive member 21 when the plunger rod 108 reaches its final position.

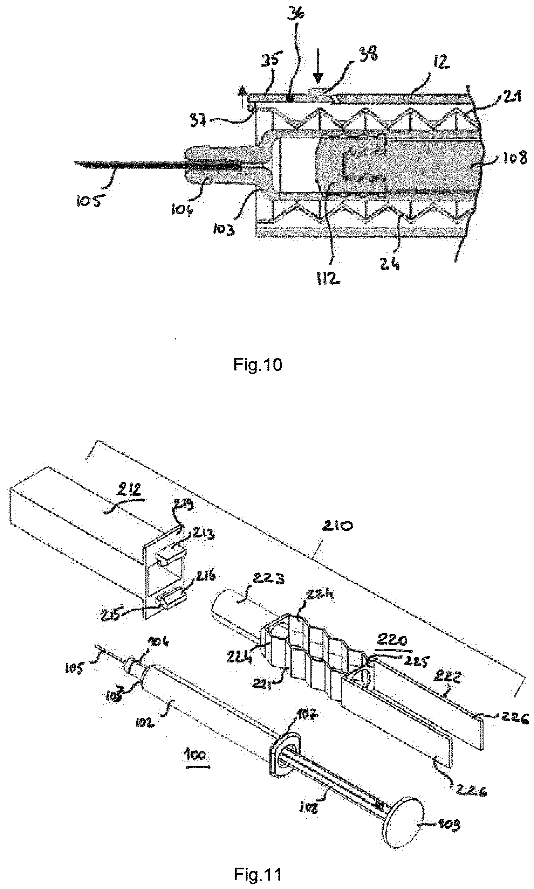

In some embodiments depicted on Fig. 9, the compressive member 21 is provided with at least one teeth 30, which extends outwardly radially from the compressive member 21 and the tubular body 12 is provided with at least one complementary teeth 31 which is configured to abut against the teeth 30. As the plunger rod 108 moves in the distal direction, the compressive member 21 is compressed against the teeth 31. The compressive member 21 is configured and sized so that it acquires an amount of mechanical energy when the plunger rod 108 reaches its final position, said amount of mechanical energy making it possible to overcome the teeth 31 thereby pushing the compressive member 21 outside of the tubular body 12 towards its safety position where the compressive member 21 covers the needle 105. In some embodiments depicted on Fig.10, the locking arrangement of the compressive member 21 relative to the tubular body 12 includes an active locking arrangement, which is actionable by a user when the injection is completed. The locking mechanism can include a tilting tab 35, which can pivot about an axis 36. The tilting tab 35 includes an inwardly oriented locking lug 37 at its distal end and an outwardly oriented pushbutton 38. The locking arrangement can suitably include an elastic component such as a pin spring (not shown) which biases the tilting tab 35 in a locked position where the locking lug 37 locks the compressive member 21 inside of tubular member 12.

After an injection is completed, the user can press the pushbutton 38; this causes the tilting tab 35 to rotate about the axis 36 and releases the locking lug 37 from the compressive member 21. In its tilted release position, the tilting tab 35 opens the way for compressive member 21 toward its extended position.

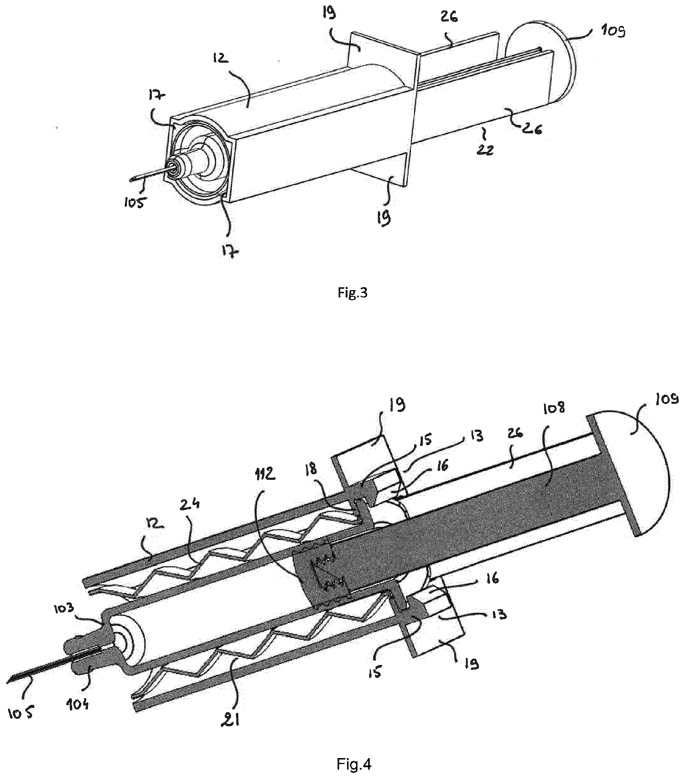

The compressive member 21 is no longer restrained. As a result, the compressive member 21 can slide distally to its extended position and shields the needle 105 in a safety state of the safety device 10. Accordingly, a user is provided with a mechanism to protect from an accidental needle stick as it can be seen on Fig.3.

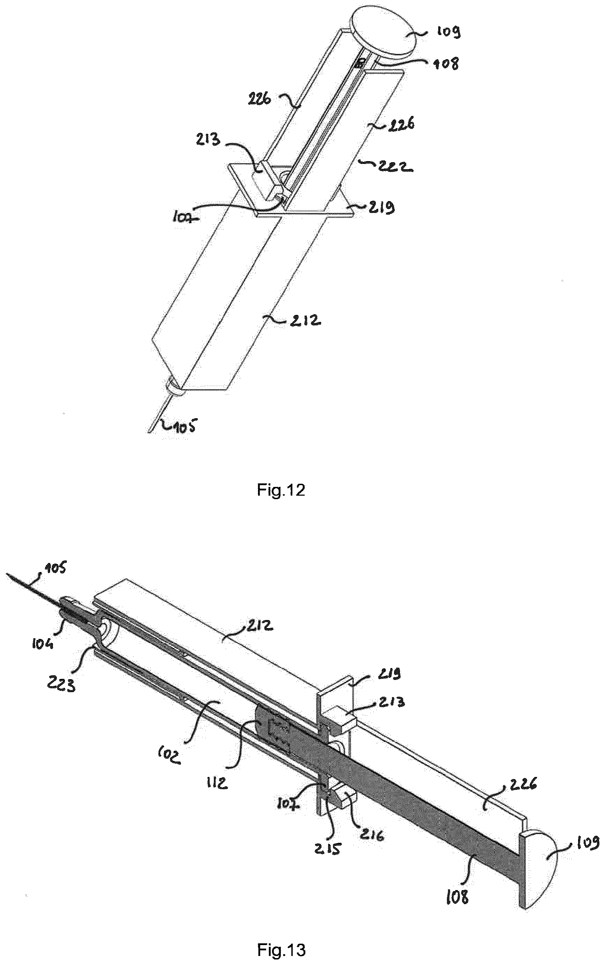

Referring to Figs.11 to 16, another embodiment of a safety member 210 is shown.

The safety device 210 includes a tubular body 212 and a safety member 220.

The tubular body 212 may include a finger flange 219 extending radially outwardly. Said tubular body 212 can be axially locked onto the drug delivery device flange 107. Thus, in some embodiments, the safety device 210 can include two hook members 213 axially and proximally projecting from the *plate 225*; each hook member 213 being comprised of a resilient tab 215 and a sloped ramp surface 216. In some embodiments, the tubular body 12 can have a square cross section configured and sized to accommodate the barrel 102.

The safety member 220 includes a compressive member 221 connected, on its proximal end, to an actuation member 222 and, on its distal end, to a tubular shield 223. The compressive member 221 can includes two strips of compressive bellows 224. The compressive member 221 can have an extended position when it is under no axial force and a collapsed position when it is under an axial force. The compressive member 221 can store mechanical energy when it is compressed under an axial force and can resiliently give back the mechanical energy when the force ceases.

The actuation member 222 includes two blades 226 are axially aligned with the compressive strips 221. A plate 225 connects the blades 226 and the strips of compressive bellows 224.

The safety member 222 may be manufactured by a molding process such as injection molding process using a thermoplastic and/or thermoplastic (TPE) or other disposable and/or recyclable material or combination thereof. Other suitable manufacturing processes may be utilized such as 3D printing.

The safety device 210 can be fitted on the drug delivery device 100. This can be done by inserting the drug delivery device 100 in the safety device 210. As the engagement progresses, the barrel flange 107 comes in abutment with the hook members 213. As the barrel flange 107 glides on the sloped ramp surfaces 216 making the tabs 215 deflect radially; this allows the barrel flange 107 to snap fit between the finger flange 219 and the hook members 213.

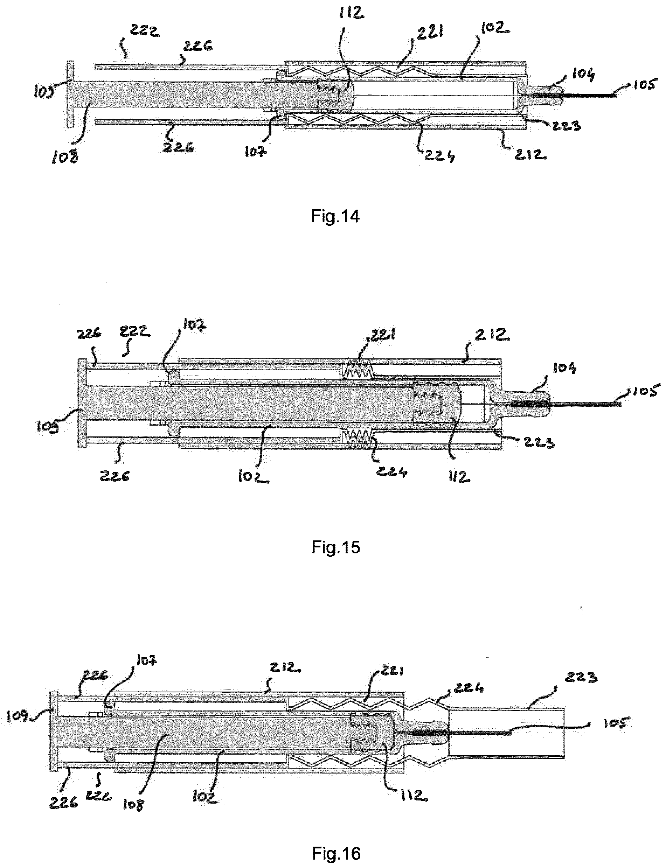

Fig.14 shows the safety device 210 in a resting state mounted on a drug delivery device 100 ready to inject a drug contained in the barrel 102.

In Fig.15, the plunger rod 108 is shown moving against the fluid (not shown). As this movement occurs, the piston flange 109 abuts against the blades 226. The blades 226 thus moves axially in the distal direction and bias the compressive member 221.

The compressive member 221 is retained by the locking arrangement which can be of the passive or active type.

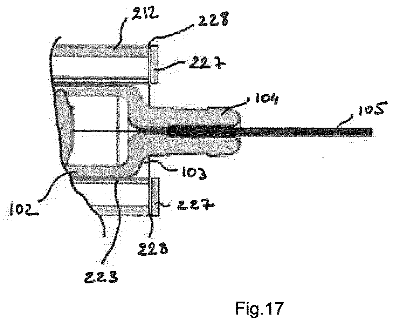

In an embodiment illustrated on Fig.17, the safety device includes a passive locking arrangement.

The tubular body 212 includes one or more locking tabs 227. The locking tabs 227 are integral with the tubular body distal end and are inwardly radially oriented. The locking tabs 227 can be connected to the tubular body by a frangible link 228, which breaks when a predetermined force is applied. In the illustrated embodiment, the tubular body 212 is provided with two locking tabs 227.

As the plunger rod 108 moves in the distal direction, the compressive member 221 is compressed against locking tabs 227. The frangible links 228 are configured and sized to break when the plunger rod 108 reaches its final position. The amount of mechanical energy accumulated in the compressive member 221 causes a force, which breaks the frangible link 228. Thus, the compressive member 221 is pushed outside of the tubular body 12 towards its safety position where the compressive member 221 covers the needle 105.

In another embodiment (not shown), the locking tabs 228 flex or elastically deflect to disengage the compressive member 221 when the plunger rod 108 reaches its final position.

In some embodiments, the safety device 210 includes an active locking arrangement such as for example the one shown on Fig.10.

The compressive member 221 is no longer restrained. As a result, the compressive member 221 can slide distally to its extended position. In the extended position of the compressive member 221, the tubular shied 223 encloses the needle 105 in a safety state of the safety device 10. Accordingly, a user is provided with a mechanism protecting from an accidental needle stick as it can be seen on Fig.16.

The present design eliminates the need to have any metal part and provides an easy to manufacture and easy to use safety device.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that changes and modifications may be made within the scope of the appended claims.

## Claims

1. A safety device (10,210) for mounting onto a drug delivery device (100) having a barrel (102) including with a flange (107) at its proximal end and an injection needle (105) at its distal end, and a piston rod (108) having a piston flange (109), the safety device (10,210) including:
a tubular body (12) configured to connect to the barrel (102),
a safety member (20, 220) at least partially disposed within the tubular body (12) configured to occupy a resting state where the safety member (20,220) does not extend distally from the tubular body (12) and a safety state where the safety member (20,220) extend distally and shields the needle (105), said safety member (20,220) having an actuation member (22, 222) configured to be pushed by the piston flange (109) and a compressive member (21,221) connected to the actuation member (22,222), said compressive member (21,221) configured to have a spring action,
a locking arrangement configured to maintain the compressive member (21, 221) within the tubular body (12) when the piston flange (109) exerts a force on the actuation member (22,222) and configured to axially release the compressive member (21,221) so that the safety member (20,220) can reach its safety state.

2. The safety device (10,210) of claim 1, wherein the actuation member (22,222) includes at least one blade (26,226) rigidly connected to the compressive member (21,221).

3. The safety device (10,210) of claim 1 or claim 2, wherein the safety device (10,210) includes at least one hook member (13,213) axially and proximally projecting from the tubular body (12,212).

4. The safety device (10,210) of claims 3, wherein the safety device (10) includes an internal rib (18), which radially projects from the tubular body (12).

5. The safety device (10,210) of one of claims 1 to 4, wherein the at least one hook member (13) being comprised of a resilient tab (15) and a sloped ramp surface (16).

6. The safety device (10,210) of one of claims 2 to 5, wherein the tubular body (12) includes at least one axial groove (17) configured to guide the at least one blade (26,226).

7. The safety device (10,210) of one of claims 1 to 6, wherein the compressive member (21) includes a series of compressible tubular bellows (24).

8. The safety device (10,210) of one of claims 1 to 7, wherein the compressive member (221) includes at least one strip of compressive bellows (226).

9. The safety device (10,210) of one of claims 1 to 8, wherein the safety member (20; 220) includes a tubular shield (223) connected to the distal end of the compressive member (21,221).

10. The safety device (10,210) of one claims 1 to 9, wherein the safety members (20,220) includes a passive locking arrangement which releases the compressive member (21, 221) when the piston rod (108) has reached its final distal position.

11. The safety device (10,210) of claim 10, wherein the safety device (10,210) includes at least one locking tab (27, 227) connected to the tubular body (12) by a frangible link (28,228) configured and sized to break when the piston rod (108) reaches its final distal position and compresses the compressive member (21,221) so that the energy accumulated in the compressive member (21,221) breaks the frangible link (28,228).

12. The safety device (10,210) of claim 10, wherein the compressive member (21) includes a least one radially outwardly oriented teeth (30) and the tubular body (12) includes a complementary inwardly oriented teeth (31) configured and sized to abut against the compressive member teeth (30) and retain the compressive member (21) until the energy accumulated in the compressive member (21) makes the compressive member teeth (30) overcome the tubular body teeth (31).

13. The safety device (10,210) of claim 10, wherein the tubular body (12) includes at least one inwardly oriented projection (14) which configured and sized to retain the compressive member (21) until the energy accumulated in the compressive member (21) makes the compressive member (21) overcome at least one projection (14).

14. The safety device (10,210) of claim 1 to 10, wherein the safety member (20,220) includes a user actionable locking arrangement which releases the compressive member (21, 221) when the piston rod (108) has reached its final distal position.

15. The safety device (10,210) of claim 14, wherein the safety member (20,220) includes a tilting tab (35) having an inwardly oriented locking lug (37) configured to pivot between a locking position where the locking lug (37) axially locks the compressive member (21,221) and a release position where the locking lug (37) opens the way for the compressive member (21,221).

16. A drug delivery device (100) having a barrel (102) including with a flange (107) at its proximal end and an injection needle (105) at its distal end, and a piston rod (108) having a piston flange (109) fitted with a safety device (10,210) according to one of claims 1 to 15.
